# EUROPEAN PATENT APPLICATION

(11) **EP 4 032 570 A1**
(43) Date of publication of application: **27.07.2022**
(21) Application number: 21161459.9
(22) Date of filing: 09.03.2021
(51) Int. Cl.: A61M 5/162, A61M 5/32, A61M 5/50

(54) **SYRINGE WITH SAFE DISPOSAL STRUCTURE**

(30) Priority: 25.01.2021 KR 20210010510
(71) Applicant: PoongLim Pharmatech Inc, Gunsan-city, Jeollabuk-do (KR)
(72) Inventor: CHO, Hee Min, 108-1303 Jeollabuk-do (KR); CHO, Mi Heui, 105-1901 Jeollabuk-do (KR); YUN, Jong Deok, 205-1003 Jeollabuk-do (KR); KIM, Jae Cheon, 201-160 Jeollabuk-do (KR)
(74) Representative: M. Zardi & Co S.A.

(57) **Abstract**

A syringe with a safe disposal structure and a function improvement structure is proposed. In the syringe, a locking protrusion protrudes on a needle safety protector main body, and a cover has a locking protrusion insertion groove, so that the cover is tightly locked alongside a barrel of a syringe assembly as the cover is pushed rearward while the locking protrusion and the locking protrusion insertion groove are engaged together. Accordingly, the injection is performed in a stable state without the obstruction of the shaking cover, and after the injection is completed, the syringe assembly is held by one hand and the cover of the needle safety protector is pressed against a surface such as a wall surface or a floor surface, so that the cover is bent around "V"-shaped bending grooves of the cover and the injection needle held in the cover is also bent, thereby preventing the reuse of the syringe.

## Description

### CROSS REFERENCE TO RELATED APPLICATION

The present application claims priority to Korean Patent Application No. 10-2021-0010510, filed January 25, 2021, the entire contents of which is incorporated herein for all purposes by this reference.

### BACKGROUND OF THE INVENTION

### Field of the Invention

The present disclosure relates generally to a syringe with a safe disposal structure and a function improvement structure and, more particularly, to a syringe capable of maintaining a stable locked state during using a needle safety protector used by being coupled to a syringe assembly and capable of safe disposal by simply folding a cover by pressing the cover against a wall or a floor in disposal to realize a safe disposal structure and a function improvement structure.

### Description of the Related Art

In general, syringes are classified into a general type syringe and a Luer-lock type syringe. The general type syringe includes a barrel as a main body of a syringe, a push rod with a finger grip, a plunger fastened to a front end of the push rod and pushing injection liquid in a piston manner, and an injection needle fastened to a tapered tube at a front end of the barrel, and the Luer-lock type syringe has a structure in which a separate Luer-lock connector is provided to be screwed to the front end of the barrel and a hub to which the injection needle is mounted is screwed to the Luer-lock connector.

In the Luer-lock type syringe, a main body of the syringe and a needle hub to which an injection needle is mounted are separately manufactured. The needle hub to which the injection needle is mounted is sealed and sterilized in a separate needle storage casing, and the main body consisting of a needle cap, a barrel, and a push rod and a needle safety protector are also sterilized and packaged in a kit configured as one set for storage and distribution. When the syringe is used, a sterile paper is removed from the needle storage casing, a screw portion formed on an inner surface of a Luer-lock connector formed on a front end of the syringe main body is aligned to fastening protrusions formed on both sides of a rear end of the needle hub and is turned. Then the syringe main body and the head portion are integrally fastened to each other and the needle safety protector is fastened to the needle hub of the assembled syringe assembly, thereby the assembled syringe is used in the integrally fastened state.

The general syringe to which the needle safety protector are not integrally mounted can be reused after use, and in some hospitals, transmission of injection by syringe reuse has become a social issue. In order to prevent this problem, a number of technique have been contrived to safely dispose of once used injection needles to prevent the used needles from being reused.

As an example, US Patent No. 6,582,397 B2 (June 24, 2003) is proposed. In the related art, a groove receiving a flange is formed on a rear portion of a needle hub, a base has a hole, and a housing is connected to the base to be foldable. When the housing is folded while the needle hub is mounted to the base, an injection needle is inserted into the housing and arms formed on opposite sides of the base are aligned and fitted to locking grooves with tension formed at lower portions of opposite sides of the housing. When the housing is folded, the locking grooves with tension formed at the lower portions of the opposite sides of the housing are opened, and when the arms of the base is inserted into the locking grooves, the locking grooves are locked by tension recovery force not to be reopened, so that the used injection needle can be safely disposed of with a safety device. As another example, flaps protrude to be curved inward on opposite sides of the housing, so that the flaps are closed inward when the needle hub is inserted into the housing and the needle hub are prevented from being removed when the flaps are returned to the initial position by the recovery force. Therefore, the used injection needle can be safely disposed of with the safety device.

However, in the structure as described above, although the used injection needle may be disposed of with the hub and the safety device, since the foldable needle housing of the needle safety protector connected to the needle hub by the hinge is connected while shaking without being fixed in use, the needle housing becomes an obstructive factor during injection, and after the injection, the needle housing is folded to allow the injection needle and the needle hub to be coupled to each other in the needle housing for disposal. However, since the needle housing is formed in a linear shape, it is difficult to press the needle housing against a wall surface or a desk surface to be folded, so a user necessarily holds the syringe by one hand and pushes and folds the needle housing by the other hand.

As another example, Korea Patent No. 10-1897956 "Needle assembly" is proposed. The needle assembly includes: a holder holding an injection needle and fastened to a needle protection cap at a front side thereof and to a syringe at a rear side thereof; a coupling device having an insertion hole provided coupling with a coupling portion at the center of the holder, and having locking grooves with locking steps on opposite sides of an upper portion of the coupling device; and a cover having an insertion space portion integrally connected to one side of the coupling device to be vertically rotated by a hinge, a supporting step having an open side to allow the injection needle to be inserted into the open side and to support the injection needle inserted into the insertion space portion not to be separated therefrom, and locking protrusions formed at opposite sides of a lower portion of the cover and configured to be inserted into one of the locking grooves of the coupling device, wherein when the injection needle is disposed of after use, a locking step of one of the locking protrusions is locked to the locking steps to be prevented from separation, so that the injection needle may be disposed of safely and be prevented from being reused. A coupling portion of the holder consists of a ring-shaped concave portion, and a protrusion is formed on an inner circumferential surface of the insertion hole of the coupling device to be inserted into the concave portion, so that the protrusion is forcibly inserted into and fastened to the concave portion. A cut groove is formed at one or more position of an inner surface of the insertion hole, and the coupling portion has an insertion protrusion at a position corresponding to the cut groove so that the coupling device and the holder are fastened to each other to be prevented from rotation.

However, in the related art configured as described above, when the needle hub is fastened to the Luer-lock type syringe main body by screwing, the cut groove is formed on the inner surface of the insertion hole of the holder, and the holder has the insertion protrusion on the position corresponding to the cut groove. When a locking protrusion of the holder is fastened to the Luer-lock screw portion of the syringe main body by screwing, the fastening is precisely performed without rotation at the fastening end point. However, as described in US Patent No. 6,582,397 B2, since the cover is connected to the coupling device by the hinge while shaking without a fixing means, the needle housing becomes an obstructive factor during injection. After the injection, the needle housing is necessarily folded to allow the injection needle and the needle hub to be coupled to each other in the needle housing for disposal. However, as described in US Patent, since the cover is formed in a linear shape, it is difficult to press the cover against a wall surface or a desk surface to be folded, so a user necessarily holds the syringe by one hand and pushes and folds the cover by the other hand, which is discomfort.

### Documents of Related Art

(Patent Document 1) US Patent No. 6,582,397 B2 (June 24, 2003);
(Patent Document 2) Korean Patent No. 10-1897956 (September 06, 2018).

### SUMMARY OF THE INVENTION

Accordingly, the present disclosure has been made keeping in mind the above problems occurring in the related art, and the present disclosure is intended to propose a syringe with a safe disposal structure and a function improvement structure. The syringe is configured to maintain a stable locked state during using a needle safety protector that is used while being integrally fastened to a needle assembly, and the syringe is configured to simply fold a cover by pressing the cover against a surface such as a wall surface or a floor surface for safe disposal after the injection.

In order to achieve the above objective, according to one aspect of the present disclosure, there is provided a syringe with a safe disposal structure and a function improvement structure. The syringe of the present disclosure may be used by integrally fastening a syringe assembly and a needle safety protector. In order to safely lock a cover of the needle safety protector, a locking protrusion may be formed by protruding on a center portion of a rear surface of a needle safety protector main body, and a locking protrusion inserting groove may be formed on the cover at a position corresponding to the locking protrusion. Wherein as the cover is pushed rearward while the locking protrusion and the locking protrusion insertion groove are engaged to each other, the cover may be tightly locked alongside a barrel of the syringe assembly. "V"-shaped bending grooves may be formed at opposite sides of a lower portion of a longitudinal center portion of the cover, so that when the used syringe assembly is pressed against a wall surface of a desk surface for disposal, the cover may be folded by the V"-shaped bending grooves and an injection needle may be bent, so that the syringe assembly may be safely discarded in a non-reusable state.

According to the present disclosure, during the injection performed while the syringe assembly and the needle safety protector are integrally fastened to each other, the locking protrusion protrudes on the center portion of the rear surface of the needle safety protector main body, and the cover has the locking protrusion insertion groove at the position corresponding to the locking protrusion, so that the cover can be tightly locked alongside the barrel of the syringe assembly as the cover is pushed rearward while the locking protrusion and the locking protrusion insertion groove are engaged to each other. Accordingly, the injection can be performed in a stable state without the obstruction of the shaking cover, and after the injection is completed, when the syringe assembly is held by one hand and the cover of the needle safety protector is pressed against a surface such as a wall surface or a floor surface, the cover is bent around the "V"-shaped bending grooves formed on the opposite sides of the lower portion of the center portion of the cover and the injection needle held in the cover is also bent, thereby preventing the reuse of the syringe. More specifically, when an upper portion of the cover is pushed rearward around the "V"-shaped bending grooves, the injection needle held in the cover is also bent so that the syringe can be discarded in a non-reusable state.

### BRIEF DESCRIPTION OF THE DRAWINGS

The above and other objectives, features, and other advantages of the present disclosure will be more clearly understood from the following detailed description when taken in conjunction with the accompanying drawings, in which:

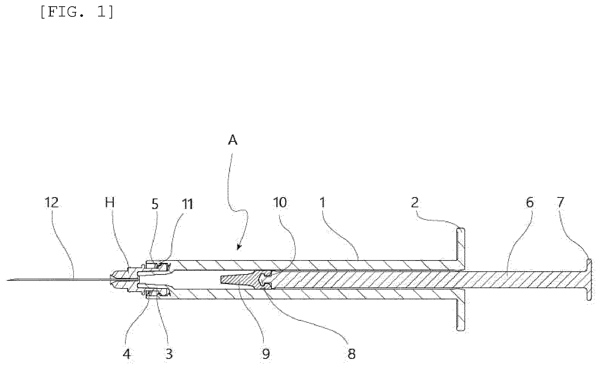

FIG. 1 is a sectional view showing a syringe assembly according to the present disclosure;

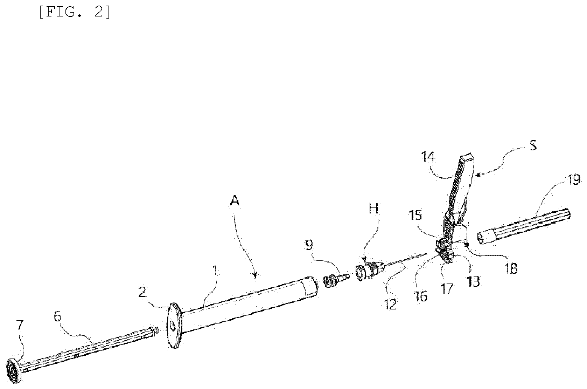

FIG. 2 is a perspective view showing a disassembled state of the syringe assembly and a needle safety protector according to the present disclosure;

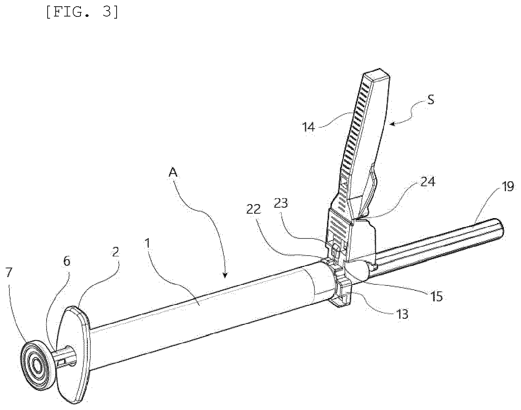

FIG. 3 is a perspective view showing an assembled state of the syringe assembly and the needle safety protector according to the present disclosure;

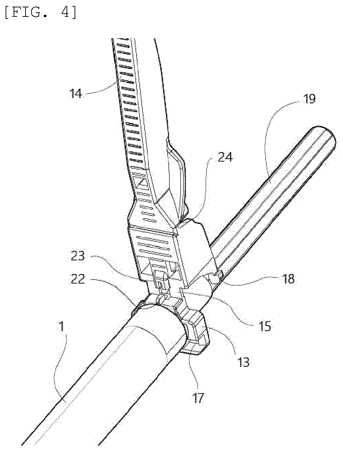

FIG. 4 is a partially enlarged perspective view showing a cover locking portion according to the present disclosure;
FIG. 5 is a perspective view showing the assembled syringe according to the present disclosure in a state in which a cover is pushed rearward and locked;

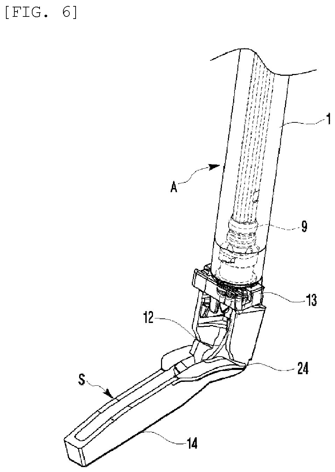

FIG. 6 is a view showing an example of bending forward the cover of the syringe according to the present disclosure for disposal after the injection; and
FIG. 7 is a view showing an example of bending the cover of the syringe according to the present disclosure by being pushed rearward for disposal after the injection.

### DETAILED DESCRIPTION OF THE INVENTION

The above and other objectives, features, and advantages of the present disclosure will be more clearly understood from the following detailed description taken in conjunction with the accompanying drawings.

In the flowing description, unless otherwise defined, all terms including technical and scientific terms used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Hereinbelow, a syringe with a safety disposal structure and a function improvement structure according to an exemplary embodiment of the present disclosure will be described in detail with reference to accompanying drawings.

As shown in the drawings, according to the present disclosure, the syringe with the safety disposal structure and the function improvement structure is configured to be used to inject with a needle safety protector S integrally fastened to a needle hub H of a syringe assembly A, and after the injection, the syringe is safely discarded with the needle safety protector S and the needle hub H integrally fastened to each other.

A barrel 1 has a finger grip 2 at a rear end thereof and a Luer-lock connector 4 in which a thread portion 5 is formed on an inner surface outside a tapered tube 3 of a front discharge end thereof, and a plunger rod 6 having a push end 7 at a rear end thereof is inserted into the barrel 1. Whereby, 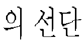 a plunger fastening protrusion 8 formed on a front end of the plunger rod 6 is fitted into a plunger fastening closed hole 10 formed inside a rear end of a plunger 9 to couple the plunger rod 6 to the plunger 9.

Meanwhile, fastening protrusion steps 11, which are formed by protruding on opposite sides of a rear end of the needle hub H to correspond to the Luer-lock connector 4 formed at the front end of the barrel 1, pass through and join to the thread portion 5, and the needle hub H to which an injection needle 12 is fastened to an end is assembled to constitute the syringe assembly A.

The needle safety protector S is fastened to the needle hub H of the syringe assembly A configured as described above. The needle safety protector S is configured such that a cover 14 is connected to one side of a needle safety protector main body 13 to be foldable by a foldable connector 15, and in order to prevent separation of the cover in the foldable state, cover fastening holes 17 are formed at both left and right sides of a needle hub fastening hole 16 formed on the center of the needle safety protector main body 13. Steps 18 protrudes outward from both sides of a lower portion of the cover 14 to be fastened to the fastening holes 17, and a locking piece 20 protrudes horizontally at one side in the cover to hold a needle cap 19 when the cover 14 is folded. Hub locking protrusions 21 protrude on both sides of the lower portion of the cover 14 to allow the needle cap 19 to be fitted into the hub locking protrusions 21, but not be separated therefrom, so that the needle cap may be safely discarded with the injection needle after use.

In the above configuration, since the cover 14 is freely connected to one side of the needle safety protector main body 13 by the foldable connector 15 during the injection, locking the cover is required.

Therefore, in order to fold and tightly lock the cover 14 alongside the barrel 1, a locking protrusion 22 protrudes on the center of a rear surface of the needle safety protector main body 13, and the cover 14 has a locking protrusion inserting groove 23 at a position corresponding to the locking protrusion 22, so that the needle safety protector main body 13 and the cover 14 are coupled and locked to each other by forced fitting.

For safe disposal after injection, "V"-shaped bending grooves 24 are formed on opposite sides of a lower portion of the center of the cover 14, so that when the cover 14 is pressed by receiving an external force, the cover 14 may be easily bent.

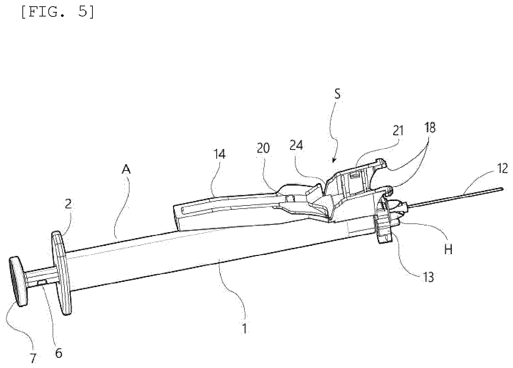

In the present disclosure configured as described above, the injection is performed while the syringe assembly A and the needle safety protector S are coupled to each other. When the cover 14 is pushed rearward before using the syringe, the locking protrusion 22 formed on the center of a rear surface of the needle safety protector main body 13 is forcibly inserted into the locking protrusion inserting groove 23 formed at a corresponding position of the cover 14. Then, the cover and the barrel 1 of the syringe are locked side by side as shown in FIG. 5, so that the injection may be safely performed without interference by the shaking of the cover 14 occurring in the injection process unless the cover is erected forward by receiving an external force.

In addition, when the cover 14 is erected forward by receiving an external force after the injection, the locking protrusion 22 of the needle safety protector 13 is separated from the locking protrusion inserting groove 23 of the cover 14 to be foldable. Then, the cover 14 is fitted to cover the injection needle 12, and when a rear surface of the cover 14 is pressed by holding the syringe assembly A by one hand and placing the syringe on a surface such as a wall surface or a floor surface, the cover 14 is bent by the "V"- shaped bending grooves 24, and at the same time, the injection needle 12 inserted in the cover is also bent, so that the syringe is prevented from being reused and the while syringe is discarded.

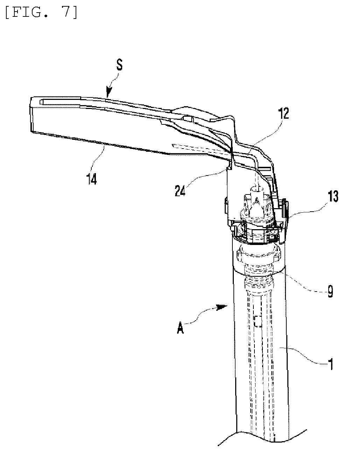

Meanwhile, in the case of bending the injection needle 12 more completely, when the cover 14 is pushed rearward as shown in FIG. 7, the cover is bent around the "V"-shaped bending grooves 24 and the injection needle 12 is completely ben and discarded.

Although the invention is described with reference to specific items such as specific structural elements, to merely some embodiments, and to drawings, such specific details disclosed herein are merely representative for purposes of helping more comprehensive understanding of the present disclosure. The present disclosure, however, is not limited to only the example embodiments set forth herein, and those skilled in the art will appreciate that the present disclosure can be embodied in many alternate forms.

Accordingly, the present disclosure is intended to cover not only the exemplary embodiments, but also various alternatives, modifications, equivalents and other embodiments that may be included within the spirit and scope of the present disclosure as defined by the appended claims.

## Claims

1. A syringe with a safe disposal structure and a function improvement structure, the syringe comprising:
a syringe assembly (A) comprising a needle hub (H); and
a needle safety protector (S) integrally assembled with the needle hub (H) of the syringe assembly (A), the needle safety protector having a cover (14),
wherein a locking protrusion (22) is formed by protruding on a center portion of a rear surface of a needle safety protector main body (13) of the needle safety protector (S) and a locking protrusion inserting groove (23) is formed on the cover (14) at a position corresponding to the locking protrusion (22), so that the cover (14) is locked while the locking protrusion (22) and the inserting groove (23) are fastened to each other.

2. The syringe of claim 1, wherein "V"-shaped bending grooves (24) are formed at opposite sides of a lower portion of a longitudinal center portion of the cover (14) of the needle safety protector (S), so that an injection needle (12) is bent by the bending grooves (24) by being pressed against a surface such as a wall surface or a floor surface to prevent the injection needle (12) after use from being reused.

3. The syringe of claim 2, wherein the cover (14) of the needle safety protector (S) is bent by being pushed rearward around the "V"-shaped bending grooves (24) to bend the injection needle (12), so that reuse of the injection needle (12) is prevented.
